# EUROPÄISCHE PATENTSCHRIFT

(11) **EP 1 935 877 B1**
(45) Veröffentlichungstag und Bekanntmachung des Hinweises auf die Patenterteilung: **22.09.2010**
(21) Anmeldenummer: 07023803.5
(22) Anmeldetag: 08.12.2007
(51) Int. Cl.: C07C 263/10, C07C 209/62, C07C 265/14

(54) **Verfahren zur Herstellung von Toluylendiisocyanat**
Process for the preparation of toluene diisocyanate
Procédé de préparation de toluène diisocyanate

(30) Priorität: 18.12.2006 DE 102006060181
(43) Veröffentlichungstag der Anmeldung: 25.06.2008
(73) Patentinhaber: Bayer MaterialScience AG, 51368 Leverkusen (DE)
(72) Erfinder: Lorenz, Wolfgang, Dr., 41540 Dormagen (DE); Padeken, Lars, Dr., 40223 Düsseldorf (DE); Pennemann, Bernd, Dr., 51467 Bergisch Gladbach (DE); Steffens, Friedhelm, 51373 Leverkusen (DE); Weismantel, Lothar, Dr., 51467 Bergisch Gladbach (DE)

(56) Entgegenhaltungen:
- WO-A-00/68180
- DE-A1- 19 827 086
- US-B2- 6 630 517

## Beschreibung

Die Erfindung betrifft ein Verfahren zur Herstellung von Toluylendiisocyanat (TDI), bei dem Toluylendiamin (TDA) mit Phosgen zu TDI umgesetzt wird, das erhaltene TDI destillativ gereinigt wird, der bei der Destillation anfallende Destillationsrückstand bei Temperaturen von kleiner 230°C und absoluten Drücken von kleiner 30 bar hydrolysiert wird, und das dabei gewonnene TDA anschließend in die Umsetzung von TDA und Phosgen zurückgeführt wird.

Die Herstellung von TDI durch Phosgenierung von TDA und die anschließende destillative Aufreinigung des rohen TDI sind allgemein bekannt. All den bekannten Verfahren zur destillativen Reinigung des rohen TDI ist gemeinsam, dass neben dem gewünschten gereinigten TDI aus der Destillation ein Destillationsrückstand erhalten wird, der weiterbehandelt werden muss.

Der Stand der Technik zur Behandlung des genannten Destillationsrückstandes der TDI-Herstellung beschreibt verschiedene Verfahren. Generelle Ziele der Rückstandsbehandlung sind die Maximierung der TDI-Ausbeute, die Minimierung des Mengenanfalls an Rückstand und eine möglichst sinnvolle kostengünstige und einfache Verwertung der für den TDI-Herstellprozess nicht mehr nutzbaren Rückstandsmenge.

### Prinzipiell sind folgende Verfahren bekannt:

Grundsätzlich kann das Gemisch aus Isocyanatprodukt und Destillationsrückstand kontinuierlich oder diskontinuierlich verbrannt werden. Das Verfahren ist technisch einfach und kann zur Nutzdampferzeugung eingesetzt werden, sofern eine dafür geeignete Anlage zur thermischen Verwertung in der Nähe der Isocyanatproduktionsanlage existiert, um eine Entsorgung über eine Rohrleitungsanbindung zu gewährleisten. Der große Nachteil dieses Verfahrens ist jedoch der Ausbeuteverlust, verursacht durch Verbrennen von Produktisocyanat (TDI-freier oder annähernd TDI-freier Destillationsrückstand ist fest).

Zur Minimierung der Isocyanatausbeuteverluste kann ein Gemisch aus TDI und Destillationsrückstand in einen gerührten und beheizten Behälter überführt und mit hochsiedenden Kohlenwasserstoffen, vorzugsweise Bitumen, die unter den Destillationsbedingungen inert sind vermischt werden, um möglichst vollständig das im Rückstand noch vorhandene freie Isocyanat abzudestillieren. Der verbleibende Rückstand kann als rieselfähiger Feststoff ausgetragen werden und einer Verbrennung zugeführt werden. Nachteile diese Verfahrens sind zusätzliches Handling und Einsatz eines prozessfremden Stoffes (Bitumen) und die aufwändigere Handhabung des Rückstandsproduktes als Feststoff (EP 0548685 A2).

Ein weiteres Verfahren zur TDI Rückstandsabtrennung ist gekennzeichnet durch den Einsatz von Knetertrocknern (US 5,446,196). In diesem Verfahren werden die oben beschriebenen beheizten und gerührten Behälter durch Knetertrockner ersetzt. Durch Einsatz von zum Beispiel Bitumen wird wie im oben genannten Beispiel der verbleibende Rückstand als rieselfähiger Feststoff erhalten der als Brennstoff zum Beispiel in Zementwerken eingesetzt werden kann. Vorteil dieses Verfahrens gegenüber dem oben genannten ist eine TDI Ausbeuteerhöhung, als Nachteil können die erforderlichen höheren Investitionskosten gesehen werden, bedingt durch die aufwändigere Technik.

In der Patentliteratur beschrieben sind auch Verfahren, in denen TDI Destillationsrückstände mit anderen Reaktanden als Wasser umgesetzt werden um neben dem Einsatzamin der Phosgenierung industriell noch nutzbare Wertstoffe zu erhalten wie zum Beispiel die Umsetzung von TDI-Rückstand mit Alkanolamin (US-A-5,902,459) oder mit MDI (DE -A-4211774, US-A-3,694,323).

Die Hydrolyse von Isocyanatdestillationsrückständen, insbesondere bei der TDI-Herstellung ist ein bereits seit längerer Zeit bearbeitetes Gebiet und beispielsweise in US-A-3,128,310, US-A-3,331,876, GB-A-795,639 und DE 2703313 A1 beschrieben.

In den zitierten Verfahren werden bei erhöhtem Druck und erhöhter Temperatur flüssiger, beziehungsweise fester TDI Destillationsrückstand mit Wasser hydrolysiert. Dabei wird ein Teil des Rückstandes in das Ursprungsamin, hier TDA umgewandelt, das nach entsprechender Aufarbeitung wieder in den Phosgenierprozess zurückgeführt werden kann und damit prinzipiell zu einer TDI Ausbeuteerhöhung und Rückstandsminimierung führt. Teilweise werden zur Beschleunigung der Reaktion Basen wie Ammoniak, das eingesetzte Ursprungsamin und auch Alkalihydroxid eingesetzt. Der Prozess kann auch zweistufig, hier unter Einsatz des Ursprungamins und Wasser, geführt werden (US-A-4,654,443). Auch der Einsatz von Dampf bei der Hydrolyse von festem Rückstand ist beschrieben, wobei Temperaturen bis 400°C beansprucht werden (US-A-3,225,094). In US-A-3,636,030 wird die saure Hydrolyse von Destillationsrückständen mit nachfolgender Trocknung und teilweiser Phosgenierung zum gewünschten Isocyanat aufgezeigt. In WO 2004/108656 A1 wird die Verarbeitung von festem TDI Destillationsrückstand beschrieben, der pulverisiert, in Wasser aufgeschlämmt und mit Alkalimetallhydroxiden, beziehungsweise Karbonaten bei Drücken von 40 - 250 bar und Temperaturen von 200 - 370 °C umgesetzt wird. Erschwerend und damit nachteilig erscheint hier der Zwischenschritt der Feststoffhandhabung in einem kontinuierlichen TDI Verfahren.

Mehrstufige und damit technisch aufwendige Prozesse, beziehungsweise die Handhabung von festen Rückständen sind auch bei den Verfahren in US-A-34,99,035, US-A-4,091,009 und US-A-4,137,266 erforderlich. In DE 19827086 A1 wird ein Hydrolyseverfahren zur Rückgewinnung von TDA aus TDI Destillationsrückstand in einem kontinuierlich durchströmten, rückvermischten Reaktor in Gegenwart von Hydrolysat vorgestellt. Als rückvermischte Reaktoren werden Rührkessel, Rührkesselkaskade, eine Reaktionsmischpumpe, ein Umpumpkreislauf mit statischem Mischer und/oder Zweistoffmischdüse, ein Strahlschlaufenreaktor oder ein Strahldüsenreaktor genannt. Die Umsetzung erfolgt bei 1-50 bar und Temperaturen von 120 - 250 °C. Das aus der Hydrolyse erhaltene Amin wird wiederum der Phosgenierung zugeführt. In US 6,630,517 B2 werden eine Vorrichtung und ein Verfahren unter anderem zum Hydrolysieren von TDI Destillationsrückstand und Recycling des rückgewonnenen Toluylendiamins in den Phosgenierprozess beansprucht. Die Hydrolyse mit reinem Wasser wird bei 30 - 300 bar Reaktordruck und einer Reaktionstemperatur von 190 - 370°C beschrieben. Die Aufarbeitung des Reaktionsgemisches erfolgt nacheinander durch Entgasung (d.h. Abtrennung des entstandenen Kohlendioxids), Entwässerung und Trennung des durch die Hydrolyse erhaltenen Produktes (hier TDA) durch Destillation unter vermindertem Druck. Der Reaktionsteil besteht aus einem oder mehreren Rohrreaktoren. Ebenfalls in einem Rohrreaktor wird die Hydrolyse von unter anderem TDI Destillationsrückstand mit Wasser im kontinuierlichen Verfahren in US 6,255,529 B1 beschrieben. Die Reaktionsbedingungen werden mit 100°C oder höher bei 50 bar oder höher angegeben, Hydrolysemittel ist Wasser.

Nachteilig an den genannten Verfahren ist jedoch der teilweise hohe Aufwand bei der Rückstandsaufarbeitung, der hohe Ausbeuteverlust und der erforderliche hohe Energieverbrauch, der unter anderem durch hohe Drücke und Temperaturen bei der Hydrolyse verursacht wird.

Ausgehend von dem Stand der Technik stellt sich nun die Aufgabe ein Verfahren zur Herstellung von TDI im Verbund von Toluolnitrierung, TDA-Herstellung, TDA-Phosgenierung TDI-Aufarbeitung und Chlorrecyclisierung bereit zu stellen, mit dem eine möglichst hohe TDI-Ausbeute erzielt werden kann und der Anfall zu entsorgender Reststoffe minimiert wird.

Es wurde nun gefunden dass dieses Ziel unter Einsatz einer Hydrolyse des TDI-Destillationsrückstandes erreicht werden kann und dass der Destillationsrückstand der TDI Aufarbeitung, im Gemisch mit TDI bereits unterhalb 30 bar Reaktionsdruck und einer Temperatur von kleiner 230°C bei Basenzusatz mit Wasser in guten Ausbeuten zu TDA hydrolysiert, welches wieder dem Phosgenierprozess zugeführt werden kann.

Die Aufgabe der vorliegenden Erfindung besteht darin, ein einfaches Verfahren zur Herstellung von TDI zur Verfügung zu stellen, bei dem der Aufwand in der Rückstandsaufarbeitung und der benötigte Energieverbrauch minimiert sind und gleichzeitig der Ausbeuteverlust an TDI und der Anfall zu entsorgender Reststoffe minimiert sind.

Die Erfindung betrifft ein Verfahren zur Herstellung von Toluylendiisocyanat, bei dem
a) Toluylendiamin mit Phosgen zu rohem Toluylendiisocyanat umgesetzt wird, und
b) das rohe Toluylendiisocyanat anschließend destillativ gereinigt wird, wobei gereinigtes Toluylendiisocyanat und ein Gemisch enthaltend Toluylendüsocyanat und Destillationsrückstand erhalten wird,
c) das Gemisch enthaltend Toluylendiisocyanat und Destillationsrückstand mit Wasser bei einer Temperatur von kleiner 230°C und einem absoluten Druck von kleiner 30 bar kontinuierlich vermischt wird und bei einer Temperatur von kleiner 230°C und einem absoluten Druck von kleiner 30 bar in einem oder mehreren hintereinander geschalteten Rohrreaktoren umgesetzt wird, wobei Toluylendiamin erhalten wird, und
d) das in Schritt c) erhaltene Toluylendiamin anschließend ggf. gereinigt und zumindest teilweise in die Umsetzung in Schritt a) zurückgeführt wird.

Die Umsetzung von TDA mit Phosgen ist grundsätzlich bekannt und zum Beispiel beschrieben in Ullmann's Encyclopedia of Industrial Chemistry, 5th ed. Vol. A 19 p. 390 ff., VCH Verlagsgesellschaft mbH, Weinheim, 1991 und G. Oertel (Ed.) Polyurethane Handbook, 2nd Edition, Hanser Verlag, München, 1993, p. 60 ff. sowie G. Wegener et. Al. Applied Catalysis A: General 221 (2001), p. 303 - 335, Elsevier Science B.V.

Bevorzugt findet die Umsetzung von TDA und Phosgen in Schritt a) wie folgt statt:

Aus TDA wird durch Umsetzung mit Phosgen in Verfahrensschritt a) TDI hergestellt. Bevorzugt stammt das TDA aus der Hydrierung von Dinitrotoluol (DNT). Der Verfahrensschritt a) wird nachfolgend auch als Phosgenierung bezeichnet. Die Umsetzung erfolgt unter Bildung von Chlorwasserstoff als Nebenprodukt.

Die Synthese von Isocyanaten im Allgemeinen und von TDI im Besonderen ist aus dem Stand der Technik hinlänglich bekannt, wobei in der Regel Phosgen in einem stöchiometrischen Überschuss, bezogen auf TDA, eingesetzt wird. Üblicherweise findet die Phosgenierung in Schritt a) in der Flüssigphase statt (DE 3744001 C1, EP 0314985 A1), wobei das Phosgen und TDA in einem Lösemittel gelöst sein können. Bevorzugte Lösemittel sind chlorierte aromatische Kohlenwasserstoffe, wie beispielsweise Chlorbenzol, o-Dichlorbenzol, p-Dichlorbenzol, Trichlorbenzole, die entsprechenden Chlortoluole oder Chlorxylole, Chlorethylbenzol, Monochlordiphenyl, α- bzw. β-Naphthylchlorid, Benzoesäureethylester, Phthalsäuredialkylester, Diisodiethylphthalat, Toluol und Xylole. Weitere Beispiele für geeignete Lösemittel sind aus dem Stand der Technik bekannt. Wie außerdem aus dem Stand der Technik, z.B. WO-A-96/16028, bekannt, kann als Lösemittel für Phosgen ebenso das gebildete Isocyanat selbst fungieren. In einer anderen, bevorzugten Ausführungsform findet die Phosgenierung oberhalb des Siedepunktes des TDA, statt. Die Gasphasenphosgenierung ist z.B. in EP 570 799 A, EP 1555258 A1, EP 1526129 A1 oder DE 10161384 A1 beschrieben. Vorteile dieses Verfahrens gegenüber der ansonsten üblichen Flüssigphasenphosgenierung liegen in der Energieeinsparung, bedingt durch die Minimierung eines aufwändigen Lösemittel- und Phosgenkreislaufs.

Das TDA kann mit Phosgen in einer einstufigen oder zweistufigen oder ggf. mehrstufigen Reaktion umgesetzt werden. Dabei ist eine kontinuierliche wie auch diskontinuierliche Betriebsweise möglich.

Wird eine einstufige Phosgenierung in der Gasphase gewählt, so erfolgt die Umsetzung oberhalb der Siedetemperatur des TDA bevorzugt innerhalb einer mittleren Kontaktzeit von 0,05 bis 5 Sekunden und bei Temperaturen von 200 bis 600°C (DE 10161384 A1).

Bei der Phosgenierung in der Flüssigphase werden üblicherweise Temperaturen von 20 bis 240°C und Drücke von 1 bis ca 50 bar eingesetzt (US-A-3,544,611). Die Phosgenierung in der Flüssigphase kann einstufig oder mehrstufig durchgeführt werden, wobei Phosgen im stöchiometrischen Überschuss eingesetzt werden kann. Dabei werden die TDA-Lösung und die Phosgenlösung bevorzugt über ein statisches Mischelement vereinigt und anschließend beispielsweise von unten nach oben durch einen oder mehrere Reaktionstürme geführt, wo das Gemisch zum gewünschten Isocyanat ausreagiert. Neben Reaktionstürmen, die mit geeigneten Mischelementen versehen sind, können auch Reaktionsbehälter mit Rührvorrichtung eingesetzt werden. Außer statischen Mischelementen können auch spezielle dynamische Mischelemente Anwendung finden. Geeignete statische und dynamische Mischelemente sind aus dem Stand der Technik bekannt.

In der Regel wird die kontinuierliche Flüssigphasen-Isocyanatherstellung im industriellen Maßstab zweistufig durchgeführt. Dabei wird in der ersten Stufe im Allgemeinen bei Temperaturen von maximal 220°C, bevorzugt maximal 160°C aus Amin und Phosgen das Carbamoylchlorid sowie aus Amin und abgespaltenem Chlorwasserstoff Aminhydrochlorid gebildet. Diese erste Stufe ist stark exotherm. In der zweiten Stufe wird sowohl das Carbamoylchlorid zu TDI und Chlorwasserstoff gespalten als auch das Aminhydrochlorid zum Carbamoylchlorid umgesetzt. Die zweite Stufe wird in der Regel bei Temperaturen von mindestens 90°C, vorzugsweise von 100 bis 240°C, durchgeführt.

Nach der Phosgenierung in Schritt a), in der ein rohes Toluylendiisocyanat erhalten wird, erfolgt in Schritt b) die Abtrennung und Reinigung des bei der Phosgenierung gebildeten TDI. Dies geschieht bevorzugt dadurch, dass zunächst das Reaktionsgemisch der Phosgenierung in einen flüssigen und einen gasförmigen Produktstrom in einer dem Fachmann bekannten Weise aufgetrennt wird. Der flüssige Produktstrom, das rohe Toluylendiisocyanat, enthält im Wesentlichen TDI, das Lösemittel sowie einen geringen Teil an nicht umgesetztem Phosgen. Der gasförmige Produktstrom besteht im Wesentlichen aus Chlorwasserstoffgas, stöchiometrisch überschüssigem Phosgen, sowie geringfügigen Mengen an Lösemittel und Inertgasen, wie zum Beispiel Stickstoff und Kohlenmonoxid. Dieser gasförmige Produktstrom wird einer weiteren Aufarbeitung zugeführt, wo in der Regel Lösungsmittel, überschüssiges Phosgen und entstandenes Chlorwasserstoffgas abgetrennt werden. Lösungsmittel und überschüssiges Phosgen werden aus wirtschaftlichen Gründen der Reaktion wieder zugeführt. Der Chlorwasserstoff kann verschiedenen Einsatzmöglichkeiten zugeführt werden, wie zum Beispiel einer Oxychlorierung von Ethylen zu Ethylendichlorid oder einem Recyclingverfahren, welches Chlor in den Isocyanatprozess wieder zurückführt. Zu diesen Recyclingverfahren zählen die katalytische Oxidation von Chlorwasserstoff, z.B. nach dem Deacon-Verfahren, die Elektrolyse von gasförmigem Chlorwasserstoff sowie die Elektrolyse einer wässrigen Lösung von Chlorwasserstoff (Salzsäure). Aus WO-A-04/14845 ist ein Verfahren zur katalytischen Oxidation nach dem Deacon-Prozess und aus WO-A-97/24320 ein Verfahren zur Gasphasenelektrolyse von Chlorwasserstoff bekannt. Eine Übersicht über elektrochemische Recycling-Verfahren wird in dem Artikel "Chlorine Regeneration from Anhydrous Hydrogen" von Dennie Turin Mah, veröffentlicht in ,,12th International Forum Electrolysis in Chemical Industry - Clean and Efficient Processing Electrochemical Technology for Synthesis, Separation, Recycle and Environmental Improvement, October 11-15, 1998, Sheraton Sand Key, Clearwater Beach, FL", gegeben.

Der flüssige Produktstrom, das rohe Toluylendiisocyanat, wird in Schritt b) anschließend einer destillativen, im Allgemeinen mehrstufigen Aufarbeitung zugeführt, wobei nacheinander noch gelöstes Phosgen sowie das Lösemittel abgetrennt werden.

Die Destillation des rohen Toluylendiisocyanats in Schritt b) kann nach allgemein bekannten Methoden (wie zum Beispiel in EP-A-1413571 und US 2003/0230476 A1 beschrieben) und bevorzugt nach einer der drei im folgenden dargestellten Varianten vorgenommen werden:

### Variante 1

Die Variante 1 ist grundsätzlich beschrieben in Chem System's PERP Report for TDI/MDI (Chem Systems, Process Evaluation Research Planning TDI/MDI 98/99 S8, Tarrytown, N.Y., USA: Chem Systems 1999, pp 27-32). Darin enthält das flüssige Reaktionsgemisch nach erfolgter destillativer Phosgenabtrennung noch einen Lösungsmittelanteil von > 50 Gew.-%, vorzugsweise 55 - 65 Gew.-%. Dieses Gemisch wird der Lösungsmittelabtrennung zugeführt, wobei in einem Vorverdampfer ein Lösungsmittel-TDI-Gemisch in eine Lösungsmitteldestillationskolonne abdestilliert wird und ein flüssiger Sumpfaustrag des Vorverdampfers einer weiteren Verarbeitung, der sogenannten Rückstandsaufarbeitung zugeführt wird. Dieser Flüssigkeitsstrom enthält neben 2-10 Gew.-% Lösungsmittel ca. 5 - 20 Gew.-% Destillationssrückstand. In der Lösungsmitteldestillationskolonne wird Lösungsmittel abdestilliert und dem Prozess wieder zugeführt. Diese Destillation kann ein- oder zweistufig durchgeführt werden (US 6,803,438 B2). Das Sumpfprodukt dieser Lösungsmitteldestillation enthält neben TDI noch 15 - 25 Gew.-% Lösungsmittelanteil. Dieser Strom wird in eine sogenannte Feinkolonne geleitet in der restliches Lösungsmittel abdestilliert wird und das lösungsmittelfreie Sumpfprodukt einer Reinkolonne zugeführt wird, die im Unterdruck betrieben wird und das gereinigte verkaufsfähige Isocyanat TDI als Destillat liefert. Ein rückstandshaltiger Teilstrom aus dem Kolonnensumpf der Reinkolonne wird ebenfalls der Rückstandsabtrennung zugeführt. Die Aufgaben der Fein- und Reindestillationskolonnen können hier alternativ wie in US 2003/0230476 A1 beschrieben in einer Trennwandkolonne zusammengefasst werden, wobei ein Strom aus Leichtsiedern und Lösungsmittel, eine Fraktion reines TDI sowie ein TDI und höhersiedende Komponenten enthaltender Produktstrom als Sumpfprodukt erhalten wird. Der letztgenannte Produktstrom wird wiederum einer Destillationsrückstandsaufarbeitung zugeführt.

### Variante 2

Im Gegensatz zu Variante 1 enthält in dieser Ausführungsform das flüssige Reaktionsgemisch nach erfolgter destillativer Phosgenabtrennung noch einen Lösungsmittelanteil von < 50 Gew.-%. Dieses Gemisch wird einem Vorverdampfer zugeführt, aus dem ein Lösungsmittel-Isocyanatgemisch mit einem Lösungsmittelanteil < 50 Gew.-%, in eine Destillationskolonne bevorzugt über Kopf abdestilliert wird. Diese Destillationskolonne entspricht der Feinkolonne in Variante 1. Der flüssige Sumpfaustrag des Vorverdampfers wird einer weiteren Verarbeitung, der sogenannten Rückstandsaufarbeitung zugeführt. Dieser Flüssigkeitsstrom enthält neben 2-10 Gew.-% Lösungsmittel ca. 5-20 Gew.-% Destillationssrückstand. Das lösungsmittelfreie Sumpfprodukt der Feinkolonne wird in die Reinkolonne geleitet, die im Unterdruck betrieben wird und das gereinigte verkaufsfähige Isocyanat TDI als Destillat liefert. Ein rückstandshaltiger Teilstrom aus dem Kolonnensumpf der Reinkolonne wird ebenfalls der Rückstandsabtrennung zugeführt. Die Aufgaben dieser Fein- und Reindestillationskolonnen können hier alternativ wie in EP 1413571 A1 beschrieben in einer Trennwandkolonne zusammengefasst werden, wobei ein Strom aus Leichtsiedern und Lösungsmittel, eine Fraktion reines TDI sowie ein TDI und höhersiedende Komponenten enthaltender Produktstrom als Sumpfprodukt erhalten wird. Der letztgenannte Produktstrom wird wiederum einer Destillationsrückstandsaufarbeitung zugeführt.

### Variante 3

Die Variante 3 umfasst die in den Varianten 2 und 1 beschriebenen Destillationssequenzen, jedoch ohne den jeweils erwähnten Vorverdampfer, der einen flüssigen Sumpfaustrag mit ca. 5-20 Gew.-% Destillationsrückstand einer Rückstandsaufarbeitung zuführt. In diesem Fall wird der Anteil an Destillationsrückstand in den beschiebenen Destillationssequenzen über die flüssigen Mengenströme bis zur jeweiligen letzten TDI Reinigungskolonne mitgeführt. Dieses Verfahren ist prinzipiell ebenfalls bekannt (EP 1717223 A2). In diesem Fall erfolgt der komplette Austrag des Destillationsrückstands (Gemisch enthaltend Toluylendiisocyanat und Destillationsrückstand)zur Rückstandsaufarbeitung aus der letzten Destillationskolonne.

All den bekannten Verfahren zur destillativen Reinigung des rohen TDI in Schritt b) ist jedoch gemeinsam, dass neben dem gewünschten gereinigten TDI aus der Destillation ein Gemisch enthaltend Toluylendiisocyanat und Destillationsrückstand erhalten wird, das weiterbehandelt werden muss.

Die Hydrolyse des Destillationsrückstands in Schritt c) erfolgt indem das Gemisch enthaltend Toluylendiisocyanat und Destillationsrückstand mit Wasser bei einer Temperatur von kleiner 230°C und einem absoluten Druck von kleiner 30 bar kontinuierlich vermischt wird (bevorzugt in einer statischen oder dynamischen Mischeinheit oder mittels Düsen, Lochblenden etc.) und bei einer Temperatur von kleiner 230°C und einem absoluten Druck von kleiner 30 bar in einem oder mehreren hintereinander geschalteten Rohrreaktoren umgesetzt wird, wobei Toluylendiamin erhalten wird. Die eingesetzten Rohrreaktoren werden dabei rückvermischungsfrei betrieben und so eine einheitliche Verweilzeitverteilung gewährleistet.

Bevorzugt wird die Hydrolyse des Destillationsrückstands wie folgt durchgeführt:

Der in den Aufarbeitungsvarianten 1 und 2 des Schrittes b) beschriebene Sumpfaustrag des Vorverdampfers enthält neben TDI und Destillationsrückstand bevorzugt 2,0 bis 10 Gew.-% Lösungsmittel. Dieser Strom wird bevorzugt über eine Destillationskolonne mit Umlaufverdampfer geführt um das restliche Lösungsmittel, welches der TDI-Herstellung wieder zugeführt werden kann, abzudestillieren. Das verbliebene TDI-Rückstandsgemisch wird bevorzugt über einen weiteren Wärmetauscher, vorzugsweise einen Fallfilmverdampfer geleitet, um das gewünschte Mengen-Verhältnis von TDI und Rückstand einzustellen.

Wird das zu hydrolysierende Gemisch enthaltend TDI und Destillationsrückstand aus der Destillation nach Variante 3 aus der letzten Destillationskolonne als Flüssigstrom entnommen, ist die oben beschriebene zusätzliche Lösungsmittelrestabtrennung nicht erforderlich. Das Gemisch enthaltend Toluylendiisocyanat und Destillationsrückstand wird dann bevorzugt direkt mittels zwangsfördernder Pumpen in den Hydrolysereaktor, d.h. einen oder mehrere hintereinander geschaltete Rohrreaktoren, gefördert.

Unabhängig von dem in Schritt b) eingesetzten Destillationsverfahren und ebenfalls unabhängig von der ggf. notwendigen Lösungsmittelabtrennung enthält das in Schritt c) in den Hydrolysereaktor eingebrachte Gemisch enthaltend Toluylendiisocyanat und Destillationsrückstand bevorzugt 10 bis 90 Gew.-%, besonders bevorzugt 40-60 Gew.-% an Toluylendiisocyanat, bezogen auf das Gewicht des Gemisches. Das in Schritt c) in den Hydrolysereaktor eingebrachte Gemisch enthaltend Toluylendiisocyanat und Destillationsrückstand enthält ebenfalls bevorzugt weniger als 1 Gew.-% an Lösungsmittel, besonders bevorzugt 0,001 bis 0,5 Gew.-% an Lösungsmittel.

Gleichzeitig wird Wasser (bevorzugt nach Vorerhitzen in einem Wärmetauscher) in den Hydrolysereaktor gefördert. Bevorzugt wird dabei das Gemisch enthaltend Toluylendiisocyanat und Destillationsrückstand und Wasser im Gewichtsverhältnis von 0,1 : 1 bis 1 : 1 eingesetzt. Bevorzugt wird als Wasser frisches Wasser, abdestilliertes Wasser aus Schritt c) und / oder bei der Dinitrotoluol (DNT) - Hydrierung entstandenes, abdestilliertes Reaktionswasser eingesetzt (EP 1484312 A1). Auch der Einsatz von Wasserdampf ist möglich. Hier zeigen sich im Verbund von Toluolnitrierung, TDA-Herstellung, TDA-Phosgenierung TDI-Aufarbeitung und Chlorrecyclisierung nutzbare Synergien, indem gereinigtes Reaktionswasser aus der DNT-Hydrierung (EP-A-236839) zur Hydrolyse eingesetzt werden kann.

Weiterhin enthält das in Schritt c) eingesetzte Wasser bevorzugt Basen. Bevorzugte eingesetzte Basen sind wässrige Natrium- oder Kaliumhydroxidlösung, meta-Toluylendiamin, ortho-Toluylendiamin, aminhaltiger Rückstand aus einer Toluylendiamin-Reindestillation, Ammoniak oder in der Dinitrotoluolhydrierung entstehende Nebenverbindungen wie zum Beispiel Toluidine, Aminomethylcyclohexane oder Diaminomethylcyclohexane. Üblicherweise enthält der Destillationsrückstand in geringen Mengen chlorierte anorganische und/oder organische Verbindungen mit Konzentrationen von bevorzugt kleiner als 1,5 Gew.-% Chlor, bezogen auf das Gewicht des Gemisches. Bei diesen Nebenkomponenten handelt es sich im Wesentlichen um Chlorwasserstoff sowie um seitenkettenchlorierte und kernchlorierte Isocyanate, daneben können noch in Spuren Verbindungen wie aromatische Isocyaniddichloride auftreten. Die Herkunft des Chlors in diesen Nebenreaktionen ist in Chlorspuren im eingesetzten Phosgen beziehungsweise in Phosgennebenreaktionen zu suchen.

Bevorzugt werden die mit dem Wasser zugeführten Basen im stöchiometrischen Überschuss zu dem in den chlorierten anorganischen und/oder organischen Verbindungen enthaltenen Chlor eingesetzt, die in dem Gemisch enthaltend Toluylendiisocyanat und Destillationsrückstand enthalten sind. Zum einen dient die Base zur Chloridbindung, zum anderen wirkt die Base als Hydrolysekatalysator und lässt im Vergleich zu den Literaturbeispielen, die in Abwesenheit von Basen durchgeführt wurden, (z.B. US 6,255,529 B1) relativ milde Reaktionsbedingungen zu, die als günstig im Hinblick auf Energieverbrauch und Apparateanforderungen einzustufen sind.

Die Verweilzeit der Umsetzung in Schritt c) beträgt bevorzugt bis zu 50 Minuten, besonders bevorzugt 2 bis 40 Minuten.

Bevorzugt entsteht in der Umsetzung in Schritt c) ein Reaktionsgemisch enthaltend Wasser, Toluylendiamin, Hydrolyserückstand und ggf. beispielsweise Natriumsalze wie Natriumchlorid und Natriumcarbonat. Bevorzugt wird das aus dem einen oder dem letzten der hintereinander geschalteten, in Schritt c) eingesetzten Rohrreaktoren austretende Reaktionsgemisch nach Vorwärmung des Einsatzwassers in einem im Gegenstrom geführten Wärmetauscher zunächst entspannt, wobei ein flüssiges Hydrolysegemisch und ein Gasgemisch erhalten werden. Die entstandenen Reaktionsgase können einer geeigneten Entsorgung, z.B. Abgasverbrennungsanlage zugeführt werden oder nach entsprechender Reinigung, wie zum Beispiel in Aktivkohleabsorptionstürmen, an die Atmosphäre abgegeben werden.

Der destillativen Gewinnung von TDA aus dem Hydrolysegemisch in Schritt d) geht bevorzugt die destillative Abtrennung von Wasser voraus. Die Abtrennung von Wasser und die Gewinnung von TDA aus dem Hydrolysegemisch können aber auch in einem gemeinsamen Destillationsschritt erfolgen.

Die Destillation in Schritt d) kann in einer separaten Sequenz von Destillationsschritten erfolgen, in der bevorzugt zunächst Wasser abgetrennt wird und anschließend TDA destillativ gewonnen wird. Die Destillation in Schritt d) kann aber auch in einer ohnehin vorhandenen Destillation durchgeführt werden, beispielsweise in einer in der TDA-Herstellung vorhandenen Destillationseinheit.

In einer bevorzugten Ausführungsform wird in Schritt d) aus dem erhaltenen flüssigen Hydrolysegemisch zunächst das überschüssige Wasser abdestilliert. Dazu wird das Hydrolysegemisch bevorzugt einer Entwässerungskolonne mit Umlaufverdampfer und gegebenenfalls einem oder mehrerer Nachverdampfer zur Restentwässerung zugeführt. Restliches noch verbliebenes Kohlendioxid wird in dieser Kolonne über Kopf abgetrennt. Das abdestillierte Wasser wird kondensiert und der Kolonne teilweise wieder als Rücklauf aufgegeben um ggf. mitgerissenes TDA zurückzuhalten. Der Wasserrücklauf wird durch einen Behälter geführt, der als Scheideflasche dient um ggf. entstehende leichtsiedende Aminkomponenten, wie zum Beispiel Diaminomethylcyclohexan, abzuscheiden. Je nach Reaktionführung der Hydrolyse kann das Amin als Base wieder eingesetzt werden oder zusammen mit den in der TDA-Entwässerung des TDA Betriebes im Isocyanatverbund abgetrennten Leichtsiedern zwecks Entsorgung vereinigt werden.

Gegebenenfalls können die in der Entwässerungsstufe der Hydrolyse erhaltenen Leichtsieder mit anderen aminhaltigen Rückständen im Isocyanatverbund, zum Beispiel mit TDA-Rückstand aus der TDA-Rückstandsentfernung zwecks Verwertung vereinigt werden.

Der in der Entwässerungskolonne erzeugte gereinigte Wasserstrom kann als Abwasser abgeführt werden oder zumindest teilweise als Hydrolysewasser in die Hydrolyse in Schritt c) zurückgeführt werden.

In Schritt d) wird aus dem Hydrolysegemisch, das bevorzugt vorab entwässert worden ist, Toluylendiamin durch Destillation erhalten und zumindest teilweise in die Umsetzung in Schritt a) zurückgeführt. Bevorzugt erfolgt die Destillation im Vakuum, d.h. bei reduziertem Druck.

Bevorzugt wird dann ein Gemisch enthaltend 70 - 90 Gew.-% TDA und 30 - 10 Gew.-% Hydrolyserückstand, bezogen auf das Gewicht des Gemisches, bei einer Temperatur von 180 - 220 °C in eine Destillationskolonne (TDA-Reinigungskolonne), die bevorzugt zur destillativen Reinigung von aus der Hydrierung von DNT erhaltenem TDA dient, gefördert. Bevorzugt wird die Destillationskolonne bei einem absoluten Druck von < 80 mbar betrieben. Die Destillationskolonne kann mit einem außen liegenden Umlaufverdampfer, der mit hochgespanntem Dampf versorgt wird, betrieben werden. Das TDA wird über den Kolonnenkopf abdestilliert und teilweise als Rücklauf der Kolonne wieder aufgegeben. Der andere Teil wird einer TDA Lagerung zugeführt. In jedem Fall wird das in Schritt c) durch Hydrolyse entstandene TDA zumindest teilweise anschließend in die Phosgenierung von TDA in Schritt a) zurückgeführt.

Der aus der Destillation in Schritt d) resultierende Hydrolyserückstand enthält üblicherweise noch 5-20 Gew.-% TDA, bezogen auf das Gewicht des Gemisches, und wird bevorzugt dem Sumpf der TDA Reinigungskolonne entnommen. Dieser Hydrolyserückstand wird anschließend bevorzugt einer Entsorgung zugeführt, die vorzugsweise einer thermischen Nutzung innerhalb des Isocyanatverbundes Toluolnitrierung, TDA-Herstellung, TDA-Phosgenierung TDI-Aufarbeitung und Chlorrecyclisierung dient. Bevorzugt werden die im Rahmen der TDA- und TDI-Herstellung anfallenden Rückstandskomponenten, im wesentlichen basierend auf flüssigen Aminkomponenten, wie zum Beispiel TDI-Hydrolyserückstand, Rückstand der TDA-Reindestillation, oder in der Dinitrotoluolhydrierung entstehende Nebenverbindungen wie zum Beispiel Toluidine, Aminomethylcyclohexane oder Diaminomethylcyclohexane und ggf. nicht genutztes ortho-TDA gemeinsam einer thermischen Nutzung (z.B. durch Verbrennung und anschließende Dampferzeugung oder Aufheizung von Stoffströmen) zugeführt. Verfahren zur katalytischen Hydrierung von Dinitrotoluol zu TDA und dessen anschließende destillative Reinigung sind dem Fachmann bekannt (z.B. EP-A-223 035 und EP-A-1 602 640). Darin liegt ein Vorteil dieses Hydrolyseverfahrens. Denn dadurch wird ermöglicht, dass in der gesamten Produktionskette TDA und TDI nur die Handhabung eines Gesamtrückstandes erforderlich ist, was praktisch eine wesentliche Vereinfachung des Verfahrens bedeutet.

Die Vorteile des erfindungsgemäßen Verfahrens können wie folgt zusammengefasst werden:

Der Anfall an Abfallprodukten aus der TDI-Herstellung ist minimiert.

Das erfindungsgemäße Verfahren ist einfach und wirtschaftlich durch die Aufarbeitung des TDI-Destillationsrückstands in einem oder mehreren einfachen Rohrreaktoren, die bei geringen Temperaturen und Drücken betrieben werden können und somit auch sicherheitstechnisch vorteilhaft betrieben werden können.

Im Vergleich zu anderen industriell eingesetzten Verfahren des Standes der Technik entfällt zum einen das Feststoffhandling von festem Destillationsrückstand und wird gleichzeitig der in Kauf genommene Verlust von Produkt durch Verbrennung vermieden.

Durch den bevorzugten Einsatz von Basen wird ein Chloreintrag in das Produkt vermieden. Der Baseneinsatz ermöglicht prinzipiell die Abtrennung rückstandshaltiger Ströme aus verschiedenen Stufen der TDI-Aufarbeitung, sodass auch in dieser Hinsicht ein Ausbeuteoptimum ermöglicht wird.

### Beispiel

TDI, das durch Phosgenierung von TDA in o-Dichlorbenzol (ODB) als Lösungsmittel hergestellt wird, wird destillativ gereinigt. Dazu wird aus einer Vorverdampferstufe der Lösungsmitteldestillation eine Flüssigphase gewonnen und diese mit dem Sumpfaustrag einer TDI-Reinigungskolonne, der sogenannten Reinkolonne, vereinigt. Die beiden Ströme weisen folgende Zusammensetzung auf:
Sumpfaustrag Vorverdampfer: 87,5 Gew.-% m-TDI, 10 Gew.-% Destillationsrückstand, 2,5 Gew.-% o-Dichlorbenzol.
Sumpfaustrag Reinkolonne: 99,98 Gew.-% m-TDI, 0,02 Gew.-% Destillationsrückstand.

Die beiden Ströme werden auf den Kopf einer Strippkolonne aufgegeben, die mit einem dampfbetriebenen Umlaufverdampfer versehen ist. In der Kolonne wird restliches ODB über Kopf abgetrennt und der Phosgenierung von TDA wieder zugeführt. Dieser abgetrennte Brüdenstrom setzt sich aus 6 Gew.-% ODB und 94 Gew.-% m-TDI zusammen. Die Sumpfentnahme dieser Kolonne ist lösungsmittelfrei und setzt sich aus 92 Gew.-% m-TDI und 8 Gew.-% Destillationsrückstand zusammen. Sodann wird dieser Strom in einen unter Vakuum betriebenen Fallfilmverdampfer überführt, der den Rückstand in diesem Gemisch weiter aufkonzentriert. Abdestilliertes TDI wird dem Prozess wieder zugeführt, das Sumpfprodukt des Fallfilmverdampfers mit jeweils 50 Gew.-% TDI und Destillationsrückstand wird zur Rückstandshydrolysereaktion gepumpt. Ein Pufferbehälter steht zur Verfügung, in den das Gemisch enthaltend TDI und Destillationsrückstand im Falle einer Betriebsunterbrechung kurzzeitig ausgelagert und dem Hydrolyseprozess wieder zugeführt werden kann. Alternativ kann das Gemisch einer thermischen Verwertung zugeführt werden.

Das Gemisch enthaltend TDI und Destillationsrückstand wird mittels einer Kolbenmembranpumpe bei 145°C und 25 bar kontinuierlich in den Hydrolysereaktor gepumpt, der aus einem von unten nach oben durchströmten Rohrreaktor besteht. Mittels einer zweiten Kolbenpumpe wird das Hydrolysemedium Wasser in den Reaktoreintritt gepumpt und mittels einer statischen Mischeinheit mit dem Gemisch enthaltend TDI und Destillationsrückstand vereinigt. Die ebenfalls mit 25 bar und 210°C kontinuierlich zugeführte wässrige Phase stellt gegenüber der organischen Phase den 2,5-fachen Massenstrom dar und enthält zusätzlich 1,5 Gew.-% Natriumhydroxid in gelöster Form. Die Hydrolysereaktion findet druckgeregelt bei 25 bar statt, wobei nach Entspannung aus dem Reaktor nach abgeschlossener Hydrolysereaktion ein Zweiphasengemisch entweicht, das 14 Gew.-% Kohlendioxid, 3,8 Gew.-% Hydrolysat, 16 Gew.-% TDA, 65 Gew.-% Wasser und 1,2 Gew.-% eines Gemisches aus Natriumchlorid und Natriumcarbonat enthält. Der Entspannung vorgeschaltet ist ein Wärmetauscher, der zur Vorwärmung des in den Reaktor eingespeisten Wassers genutzt wird. Die Entspannung erfolgt von 25 bar auf annähernd Atmosphärendruck, wobei das Reaktionsgemisch in einen der Entwässerungskolonne vorgeschalteten Entspannungsbehälter geleitet wird.

Von diesem Entspannungsbehälter werden ein gasförmiger und ein flüssiger Strom in die Entwässerungskolonne geführt, wobei unter den gegebenen Druck- und Temperaturbedingungen von 106°C/1400 mbar absolut 42 Gew.-% gasförmig und 58 Gew.-% des Reaktionsgemisches in flüssiger Form in die Kolonne gelangen. Der gasförmige Anteil setzt sich aus 66 Gew.-% Wasser und 34 Gew.-% Kohlendioxid zusammen, während der flüssige Strom zu 65 Gew.-% aus Wasser und zu 28 % aus TDA besteht. Der Rest im Flüssigstrom bilden Hydrolysat und die genannten anorganischen Anteile.

Kohlendioxid und Wasser werden vom Sumpfprodukt TDA und Hydrolysat abgetrennt. Der Sumpfaustrag der Entwässerungskolonne enthält nach Durchlaufen des Umlaufverdampfers 1,0 Gew.-% Wasser, 75 Gew.-% TDA und 24 Gew.-% Hydrolysat mit anorganischen Anteilen bei Normaldruck und einer Temperatur von 200°C. Mit einem nachgeschalteten dampfbetriebenen Röhrenwärmetauscher wird restliches Wasser entfernt und das verbliebene Gemisch in eine Vakuumkolonne geführt, in der die Trennung des Hydrolysats von TDA bei einem absoluten Druck von 80 mbar und einer Kolonnensumpftemperatur von 273°C durchgeführt wird.

Aus der Vakuumkolonne wird gereinigtes TDA abdestilliert und der Phosgenierung wieder zugeführt. Das Hydrolysat kann als Sumpfprodukt mit einem Rest TDA-Gehalt von 8 Gew.-% einer Verwertung zugeführt werden.

Die technisch verwertbare TDA-Ausbeute in diesem Beispiel liegt bei 78 Gew.-%, bezogen auf eingesetztes hydrolysiertes TDI und TDI Rückstand (berechnet mit Molmasse von TDI).

## Patentansprüche

1. Verfahren zur Herstellung von Toluylendiisocyanat, bei dem
a) Toluylendiamin mit Phosgen zu rohem Toluylendiisocyanat umgesetzt wird, und
b) das rohe Toluylendiisocyanat anschließend destillativ gereinigt wird, wobei gereinigtes Toluylendiisocyanat und ein Gemisch enthaltend Toluylendiisocyanat und Destillationsrückstand erhalten wird, und
c) das Gemisch enthaltend Toluylendiisocyanat und Destillationsrückstand mit Wasser bei einer Temperatur von kleiner 230°C und einem absoluten Druck von kleiner 30 bar kontinuierlich vermischt wird und bei einer Temperatur von kleiner 230°C und einem absoluten Druck von kleiner 30 bar in einem oder mehreren hintereinander geschalteten Rohrreaktoren umgesetzt wird, wobei Toluylendiamin erhalten wird, und
d) das in Schritt c) erhaltene Toluylendiamin anschließend ggf. gereinigt und zumindest teilweise in die Umsetzung in Schritt a) zurückgeführt wird.

2. Verfahren nach Anspruch 1, bei dem das in Schritt b) erhaltene Gemisch enthaltend Toluylendiisocyanat und Destillationsrückstand 10 bis 90 Gew.-% an Toluylendiisocyanat, bezogen auf das Gewicht des Gemisches enthält.

3. Verfahren nach einem der Ansprüche 1 oder 2, bei dem das Gemisch enthaltend Toluylendiisocyanat und Destillationsrückstand und Wasser im Gewichtsverhältnis von 0,1 : 1 bis 1 : 1 eingesetzt werden.

4. Verfahren nach einem der Ansprüche 1 bis 3, bei dem das in Schritt c) eingesetzte Wasser Basen enthält.

5. Verfahren nach Anspruch 4, bei dem das Gemisch enthaltend Toluylendiisocyanat und Destillationsrückstand Chlor in Form von chlorierten anorganischen und / oder organischen Verbindungen enthält und die Basen im stöchiometrischen Überschuss zu dem Chlor eingesetzt werden.

6. Verfahren nach einem der Ansprüche 1 bis 5, bei dem in der Umsetzung in Schritt c) ein Reaktionsgemisch enthaltend Wasser, Toluylendiamin, Hydrolyserückstand und ggf. Chloridsalz entsteht, und bei dem das aus dem einen oder dem letzten der hintereinander geschalteten Rohrreaktoren austretende Reaktionsgemisch zunächst entspannt wird; wobei ein flüssiges Hydrolysegemisch und ein Gasgemisch erhalten werden.

7. Verfahren nach Anspruch 6, bei dem aus dem erhaltenen flüssigen Hydrolysegemisch Toluylendiamin durch Destillation, bevorzugt durch Vakuumdestillation, erhalten wird.

8. Verfahren nach Anspruch 7, bei dem aus dem erhaltenen flüssigen Hydrolysegemisch zunächst destillativ Wasser entfernt wird und anschließend das Toluylendiamin durch Destillation erhalten wird.

9. Verfahren nach Anspruch 8, bei dem das aus dem flüssigen Hydrolysegemisch destillativ entfernte Wasser in die Hydrolyse in Schritt c) zurückgeführt wird.

10. Verfahren nach Anspruch 7, bei dem bei der Destillation des flüssigen Hydrolysegemisches neben Toluylendiamin ein Gemisch enthaltend Toluylendiamin und Hydrolyserückstand erhalten wird, und das Gemisch enthaltend Toluylendiamin und Hydrolyserückstand anschließend zusammen mit Toluylendiamin enthaltenden Rückständen, die bei der katalytischen Hydrierung von Dinitrotoluol zu Toluylendiamin und/oder dessen destillativer Aufbereitung anfallen und/oder zusammen mit o-Toluylendiamin einer gemeinsamen thermischen Nutzung zugeführt werden.

## Claims

1. Process for the preparation of toluylene-diisocyanate, in which
a) toluylenediamine is reacted with phosgene to give crude toluylene-diisocyanate, and
b) the crude toluylene-diisocyanate is then purified by distillation, purified toluylene-diisocyanate and a mixture containing toluylene-diisocyanate and distillation residue being obtained, and
c) the mixture containing toluylene-diisocyanate and distillation residue is mixed continuously with water at a temperature of less than 230 °C under an absolute pressure of less than 30 bar and the mixture is reacted at a temperature of less than 230 °C under an absolute pressure of less than 30 bar in one or more tube reactors connected in series, toluylenediamine being obtained, and
d) the toluylenediamine obtained in step c) is then optionally purified and at least partly recycled into the reaction in step a).

2. Process according to claim 1, in which the mixture obtained in step b) containing toluylene-diisocyanate and distillation residue contains 10 to 90 wt.% of toluylene-diisocyanate, based on the weight of the mixture.

3. Process according to one of claims 1 or 2, in which the mixture containing toluylene-diisocyanate and distillation residue and water is employed in a weight ratio of 0.1 : 1 to 1 : 1.

4. Process according to one of claims 1 to 3, in which the water employed in step c) contains bases.

5. Process according to claim 4, in which the mixture containing toluylene-diisocyanate and distillation residue contains chlorine in the form of chlorinated inorganic and / or organic compounds and the bases are employed in a stoichiometric excess relative to the chlorine.

6. Process according to one of claims 1 to 5, in which in the reaction in step c) a reaction mixture containing water, toluylenediamine, hydrolysis residue and optionally chloride salt is formed, and in which the reaction mixture leaving the one or the last of the tube reactors connected in series is first let down, a liquid hydrolysis mixture and a gas mixture being obtained.

7. Process according to claim 6, in which toluylenediamine is obtained from the resulting liquid hydrolysis mixture by distillation, preferably by vacuum distillation.

8. Process according to claim 7, in which water is first removed from the resulting liquid hydrolysis mixture by distillation and the toluylenediamine is then obtained by distillation.

9. Process according to claim 8, in which the water removed from the liquid hydrolysis mixture by distillation is recycled into the hydrolysis in step c).

10. Process according to claim 7, in which, in addition to toluylenediamine, a mixture containing toluylenediamine and hydrolysis residue is obtained in the distillation of the liquid hydrolysis mixture, and the mixture containing toluylenediamine and hydrolysis residue is then fed together with residues which contain toluylenediamine and are obtained in the catalytic hydrogenation of dinitrotoluene to give toluylenediamine and/or working up thereof by distillation and/or together with o-toluylenediamine to a common thermal utilization.

## Revendications

1. Procédé de préparation de diisocyanate de toluylène où
a) on convertit de la toluylènediamine en diisocyanate de toluylène brut au moyen de phosgène et
b) on purifie ensuite le diisocyanate de toluylène brut par distillation, en obtenant du diisocyanate de toluylène purifié et un mélange contenant du diisocyanate de toluylène et du résidu de distillation, et
c) on mélange continûment le mélange contenant du diisocyanate de toluylène et du résidu de distillation avec de l'eau à une température inférieure à 230°C et sous une pression absolue inférieure à 30 bars et on le met à réagir à une température inférieure à 230°C et sous une pression absolue inférieure à 30 bars dans un ou plusieurs réacteurs tubulaires placés en série, ce qui permet d'obtenir de la toluylènediamine et
d) la toluylènediamine obtenue à l'étape c) est ensuite purifiée le cas échéant et recyclée au moins en partie à la conversion de l'étape a).

2. Procédé selon la revendication 1 où le mélange contenant du diisocyanate de toluylène et du résidu de distillation obtenu à l'étape b) contient 10 à 90 % en poids de diisocyanate de toluylène, par rapport au poids du mélange.

3. Procédé selon l'une des revendications 1 et 2, où le mélange contenant du diisocyanate de toluylène et du résidu de distillation et l'eau sont utilisés dans un rapport pondéral de 0,1 : 1 à 1 : 1.

4. Procédé selon l'une des revendications 1 à 3, où l'eau utilisée à l'étape c) contient des bases.

5. Procédé selon la revendication 4, où le mélange contenant du diisocyanate de toluylène et du résidu de distillation contient du chlore sous la forme de composés chlorés minéraux et/ou organiques et les bases sont utilisées en un excès stoechiométrique par rapport au chlore.

6. Procédé selon l'une quelconque des revendications 1 à 5, où il se forme au cours de la mise à réagir de l'étape c) un mélange réactionnel contenant de l'eau, de la toluylènediamine, du résidu d'hydrolyse et le cas échéant le sel chlorure et où le mélange réactionnel sortant de l'unique ou du dernier des réacteurs tubulaires en série est d'abord détendu, ce qui permet d'obtenir un mélange d'hydrolyse liquide et un mélange gazeux.

7. Procédé selon la revendication 6, où à partir du mélange d'hydrolyse liquide obtenu on obtient de la toluylènediamine par distillation, de préférence par distillation sous vide.

8. Procédé selon la revendication 7, où on chasse tout d'abord l'eau par distillation du mélange d'hydrolyse liquide obtenu et on obtient ensuite la toluylènediamine par distillation.

9. Procédé selon la revendication 8, où l'eau chassée par distillation du mélange d'hydrolyse liquide est recyclée à l'hydrolyse de l'étape c).

10. Procédé selon la revendication 7, où lors de la distillation du mélange d'hydrolyse liquide, on obtient, outre la toluylènediamine, un mélange contenant de la toluylènediamine et du résidu d'hydrolyse et le mélange contenant de la toluylènediamine et du résidu d'hydrolyse est recyclé ensuite à une utilisation thermique commune avec les résidus contenant de la toluylènediamine qui se forment lors de l'hydrogénation catalytique de dinitrotoluène en toluylènediamine et/ou de sa préparation par distillation et/ou avec la o-toluylènediamine.
